# EUROPEAN PATENT SPECIFICATION

(11) **EP 3 264 899 B1**
(45) Date of publication and mention of the grant of the patent: **12.06.2019**
(21) Application number: 16709514.0
(22) Date of filing: 02.03.2016
(51) Int. Cl.: A01N 63/04, A01P 13/00

(54) **A MYCOHERBICIDE**
MYKOHERBIZID
MYCOHERBICIDE

(30) Priority: 02.03.2015 GB 201503510
(43) Date of publication of application: 10.01.2018
(73) Proprietor: The Secretary Of State For Environment, Food & Rural Affairs, London SW1P 3JR (GB)
(72) Inventor: SEIER, Marion Karin, Ascot Berkshire SL5 9BJ (GB); EVANS, Harold Charles, Camberley Surrey GU15 4JR (GB)
(74) Representative: Arends, William Gerrit
(86) International application number: PCT/GB2016/050553
(87) International publication number: WO 2016/139476

(56) References cited:
- US-A- 5 538 890
- "CABI Annual Report - Europe UK 2013", , 1 January 2014 (2014-01-01), pages 1-76, XP055268893, Egham, UK Retrieved from the Internet: URL:http://www.cabi.org/Uploads/CABI/news/ UK annual report.pdf [retrieved on 2016-04-27]
- "Progress with Weed Biocontrol projects", CABI in the UK, 1 June 2013 (2013-06-01), pages 1-3, XP055268651, Retrieved from the Internet: URL:http://www.cabi.org/uploads/projectsdb /documents/5589/update on biocontrol projects for GB.pdf [retrieved on 2016-04-26]
- DAISUKE KUROSE ET AL: "Systematics of Mycosphaerella species associated with the invasive weed Fallopia japonica, including the potential biological control agent M. polygoni-cuspidati", MYCOSCIENCE, SPRINGER-VERLAG, TO, vol. 50, no. 3, 29 May 2009 (2009-05-29), pages 179-189, XP019665179, ISSN: 1618-2545

### SEQUENCE LISTING

<110> CABI
<120> A MYCOHERBICIDE
<130> PN817335WO
<150> GB1503510.8
   <151> 2015-03-02
<160> 18
<170> PatentIn version 3.5
<210> 1
   <211> 1296
   <212> DNA
   <213> Mycosphaerella polygoni-cuspidati
<220>
   <221> CDS
   <222> (1)..(259)
<220>
   <221> CDS
   <222> (309)..(402)
<220>
   <221> CDS
   <222> (458)..(626)
<220>
   <221> CDS
   <222> (679)..(1293)
<400> 1
   atg gct acc caa gcc ctg cca gac acc ctt gac acc ctt cgc gac ctc        48
   Met Ala Thr Gln Ala Leu Pro Asp Thr Leu Asp Thr Leu Arg Asp Leu
   1               5                   10                  15
   ttc gcg acc gtc ccc gag gag atg gtt acc cag ctc gtt tct gag gtc        96
   Phe Ala Thr Val Pro Glu Glu Met Val Thr Gln Leu Val Ser Glu Val
               20                  25                  30
   acg gct gcg ctg agc gaa gca gca gct cga cga gcc aca gca ccg aga       144
   Thr Ala Ala Leu Ser Glu Ala Ala Ala Arg Arg Ala Thr Ala Pro Arg
           35                  40                  45
   cgc acc gca cat ggc gca atg ccc ggc acg act gcc acc acc tcg gct       192
   Arg Thr Ala His Gly Ala Met Pro Gly Thr Thr Ala Thr Thr Ser Ala
       50                  55                  60
   gta gcg aag acg cag cgt gtg gcc ggt ggg cca aag aga ccg ctc aac       240
   Val Ala Lys Thr Gln Arg Val Ala Gly Gly Pro Lys Arg Pro Leu Asn
   65                  70                  75                  80
   tcc tgg atg gca ttc cgc a gtatgtccac ctccctcctt gctcttgtct            289
   Ser Trp Met Ala Phe Arg
                   85
   cttactgact tcaacacag ag  ttc tac aac ggc gcg ctc tcg ccg cac act      340
                       Lys Phe Tyr Asn Gly Ala Leu Ser Pro His Thr
                                   90                  95
   cag aaa gtg atc tcc aaa gcc ctg acg aag ttt tgg cgt gag gac ccc      388
   Gln Lys Val Ile Ser Lys Ala Leu Thr Lys Phe Trp Arg Glu Asp Pro
           100                 105                 110
   ttc ggc gcc aag tg  gttcgtaact ccatgttctt cacctgacac tgaagcatct      442
   Phe Gly Ala Lys Trp
       115
   gctaacatat cgcag g gtc att ctc gcc aag gca tac agt gtc gtg cgc       491
                      Val Ile Leu Ala Lys Ala Tyr Ser Val Val Arg
                          120                 125
   gga agc cgc gag aag caa gag gct ccg ctt gac gag ttc ttc atc atc      539
   Gly Ser Arg Glu Lys Gln Glu Ala Pro Leu Asp Glu Phe Phe Ile Ile
   130                 135                 140                 145
   tgc gca cca ctc atc ggc gtc att ccg cca aac gag tac atg cag cag      587
   Cys Ala Pro Leu Ile Gly Val Ile Pro Pro Asn Glu Tyr Met Gln Gln
                   150                 155                 160
   atg gga tgg cat ctc ggc ccg cca atg gag ggc gac aag gtaccgtgcc       636
   Met Gly Trp His Leu Gly Pro Pro Met Glu Gly Asp Lys
               165                 170
   ccccgcagcc ccttcgttgc ctcagtgctg acctctacct ag gag aag atg ccg       690
                                                  Glu Lys Met Pro
                                                  175
   cag atc act cgc ctc ttc atc cca aac ctc gac acc ttt cct gat gcg      738
   Gln Ile Thr Arg Leu Phe Ile Pro Asn Leu Asp Thr Phe Pro Asp Ala
       180                 185                 190
   atc acg atg acc aca ctc agt gtc gac gat ctg gtc cag gcc tgc tac      786
   Ile Thr Met Thr Thr Leu Ser Val Asp Asp Leu Val Gln Ala Cys Tyr
   195                 200                 205                 210
   aac gcc ggc tac gtc gac gcc agc aac gac gac ttg ccg agc gca agc      834
   Asn Ala Gly Tyr Val Asp Ala Ser Asn Asp Asp Leu Pro Ser Ala Ser
                   215                 220                 225
   acc cag ggc tca ctc acc atg gtc gcc cgg cca act ccc gtc acc aac      882
   Thr Gln Gly Ser Leu Thr Met Val Ala Arg Pro Thr Pro Val Thr Asn
               230                 235                 240
   gcc gac atg gct gtg acc aca cat gtg aca ccc gtc gct cag act gac      930
   Ala Asp Met Ala Val Thr Thr His Val Thr Pro Val Ala Gln Thr Asp
           245                 250                 255
   atc acc aac ccc gac gtc gag cca gat ctt gag cca gag ttc gag cca      978
   Ile Thr Asn Pro Asp Val Glu Pro Asp Leu Glu Pro Glu Phe Glu Pro
       260                 265                 270
   gat ttc gag cca ttc aac gac cac cgc cct gtc cca ctc gac cat ggt     1026
   Asp Phe Glu Pro Phe Asn Asp His Arg Pro Val Pro Leu Asp His Gly
   275                 280                 285                 290
   cag tac gca caa cag acc gtc acg ccc gct cag ctg tcc aac aac gag     1074
   Gln Tyr Ala Gln Gln Thr Val Thr Pro Ala Gln Leu Ser Asn Asn Glu
                   295                 300                 305
   gcc atc gag atc gtc cag tac cac ggc gac gaa gac ctc ggc ctg ttc      1122
   Ala Ile Glu Ile Val Gln Tyr His Gly Asp Glu Asp Leu Gly Leu Phe
               310                 315                 320
   ttc aac cct gtc gac ggc atg ttc ggc acc ttc ttc aac cca aac gag      1170
   Phe Asn Pro Val Asp Gly Met Phe Gly Thr Phe Phe Asn Pro Asn Glu
           325                 330                 335
   aac aac gac gaa ttc gcc gcc ttc gac atg gac atc tac aac cac gcc      1218
   Asn Asn Asp Glu Phe Ala Ala Phe Asp Met Asp Ile Tyr Asn His Ala
       340                 345                 350
   ccg cac act acc gca aac gag gtc cag cag tct gtc cag aac gcc tcc      1266
   Pro His Thr Thr Ala Asn Glu Val Gln Gln Ser Val Gln Asn Ala Ser
   355                 360                 365                 370
   ctc gac aac acc ttc tac tac gac ggt tga                              1296
   Leu Asp Asn Thr Phe Tyr Tyr Asp Gly
                   375
<210> 2
   <211> 379
   <212> PRT
   <213> Mycosphaerella polygoni-cuspidati
<400> 2
   Met Ala Thr Gln Ala Leu Pro Asp Thr Leu Asp Thr Leu Arg Asp Leu
   1               5                   10                  15
   Phe Ala Thr Val Pro Glu Glu Met Val Thr Gln Leu Val Ser Glu Val
               20                  25                  30
   Thr Ala Ala Leu Ser Glu Ala Ala Ala Arg Arg Ala Thr Ala Pro Arg
           35                  40                  45
   Arg Thr Ala His Gly Ala Met Pro Gly Thr Thr Ala Thr Thr Ser Ala
       50                  55                  60
   Val Ala Lys Thr Gln Arg Val Ala Gly Gly Pro Lys Arg Pro Leu Asn
   65                  70                  75                  80
   Ser Trp Met Ala Phe Arg Lys Phe Tyr Asn Gly Ala Leu Ser Pro His
                   85                  90                  95
   Thr Gln Lys Val Ile Ser Lys Ala Leu Thr Lys Phe Trp Arg Glu Asp
               100                 105                 110
   Pro Phe Gly Ala Lys Trp Val Ile Leu Ala Lys Ala Tyr Ser Val Val
           115                 120                 125
   Arg Gly Ser Arg Glu Lys Gln Glu Ala Pro Leu Asp Glu Phe Phe Ile
       130                 135                 140
   Ile Cys Ala Pro Leu Ile Gly Val Ile Pro Pro Asn Glu Tyr Met Gln
   145                 150                 155                 160
   Gln Met Gly Trp His Leu Gly Pro Pro Met Glu Gly Asp Lys Glu Lys
                   165                 170                 175
   Met Pro Gln Ile Thr Arg Leu Phe Ile Pro Asn Leu Asp Thr Phe Pro
               180                 185                 190
   Asp Ala Ile Thr Met Thr Thr Leu Ser Val Asp Asp Leu Val Gln Ala
           195                 200                 205
   Cys Tyr Asn Ala Gly Tyr Val Asp Ala Ser Asn Asp Asp Leu Pro Ser
       210                 215                 220
   Ala Ser Thr Gln Gly Ser Leu Thr Met Val Ala Arg Pro Thr Pro Val
   225                 230                 235                 240
   Thr Asn Ala Asp Met Ala Val Thr Thr His Val Thr Pro Val Ala Gln
                   245                 250                 255
   Thr Asp Ile Thr Asn Pro Asp Val Glu Pro Asp Leu Glu Pro Glu Phe
               260                 265                 270
   Glu Pro Asp Phe Glu Pro Phe Asn Asp His Arg Pro Val Pro Leu Asp
           275                 280                 285
   His Gly Gln Tyr Ala Gln Gln Thr Val Thr Pro Ala Gln Leu Ser Asn
       290                 295                 300
   Asn Glu Ala Ile Glu Ile Val Gln Tyr His Gly Asp Glu Asp Leu Gly
   305                 310                 315                 320
   Leu Phe Phe Asn Pro Val Asp Gly Met Phe Gly Thr Phe Phe Asn Pro
                   325                 330                 335
   Asn Glu Asn Asn Asp Glu Phe Ala Ala Phe Asp Met Asp Ile Tyr Asn
               340                 345                 350
   His Ala Pro His Thr Thr Ala Asn Glu Val Gln Gln Ser Val Gln Asn
           355                 360                 365
   Ala Ser Leu Asp Asn Thr Phe Tyr Tyr Asp Gly
       370                 375
<210> 3
   <211> 297
   <212> DNA
   <213> Mycosphaerella polygoni-cuspidati
<220>
   <221> CDS
   <222> (1)..(297)
<400> 3
   cca aag aga ccg ctc aac tcc tgg atg gca ttc cgc aag ttc tac aac        48
   Pro Lys Arg Pro Leu Asn Ser Trp Met Ala Phe Arg Lys Phe Tyr Asn
   1               5                   10                  15
   ggc gcg ctc tcg ccg cac act cag aaa gtg atc tcc aaa gcc ctg acg        96
   Gly Ala Leu Ser Pro His Thr Gln Lys Val Ile Ser Lys Ala Leu Thr
               20                  25                  30
   aag ttt tgg cgt gag gac ccc ttc ggc gcc aag tgg gtc att ctc gcc       144
   Lys Phe Trp Arg Glu Asp Pro Phe Gly Ala Lys Trp Val Ile Leu Ala
           35                  40                  45
   aag gca tac agt gtc gtg cgc gga agc cgc gag aag caa gag gct ccg       192
   Lys Ala Tyr Ser Val Val Arg Gly Ser Arg Glu Lys Gln Glu Ala Pro
       50                  55                  60
   ctt gac gag ttc ttc atc atc tgc gca cca ctc atc ggc gtc att ccg       240
   Leu Asp Glu Phe Phe Ile Ile Cys Ala Pro Leu Ile Gly Val Ile Pro
   65                  70                  75                  80
   cca aac gag tac atg cag cag atg gga tgg cat ctc ggc ccg cca atg       288
   Pro Asn Glu Tyr Met Gln Gln Met Gly Trp His Leu Gly Pro Pro Met
                   85                  90                  95
   gag ggc gac                                                           297
   Glu Gly Asp
<210> 4
   <211> 99
   <212> PRT
   <213> Mycosphaerella polygoni-cuspidati
<400> 4
   Pro Lys Arg Pro Leu Asn Ser Trp Met Ala Phe Arg Lys Phe Tyr Asn
   1               5                   10                  15
   Gly Ala Leu Ser Pro His Thr Gln Lys Val Ile Ser Lys Ala Leu Thr
               20                  25                  30
   Lys Phe Trp Arg Glu Asp Pro Phe Gly Ala Lys Trp Val Ile Leu Ala
           35                  40                  45
   Lys Ala Tyr Ser Val Val Arg Gly Ser Arg Glu Lys Gln Glu Ala Pro
       50                  55                  60
   Leu Asp Glu Phe Phe Ile Ile Cys Ala Pro Leu Ile Gly Val Ile Pro
   65                  70                  75                  80
   Pro Asn Glu Tyr Met Gln Gln Met Gly Trp His Leu Gly Pro Pro Met
                   85                  90                  95
   Glu Gly Asp
<210> 5
   <211> 1087
   <212> DNA
   <213> Mycosphaerella polygoni-cuspidati
<220>
   <221> CDS
   <222> (1)..(185)
<220>
   <221> CDS
   <222> (239)..(435)
<220>
   <221> CDS
   <222> (489)..(1084)
<400> 5
   atg acc acc agc atc acc acc cac ccc ccg cag gcg ccg ctg cca gag          48
   Met Thr Thr Ser Ile Thr Thr His Pro Pro Gln Ala Pro Leu Pro Glu
   1               5                   10                  15
   ctc gac cag ttc ttc ctg aat gag act cgc atc acc gac tac atc gtc          96
   Leu Asp Gln Phe Phe Leu Asn Glu Thr Arg Ile Thr Asp Tyr Ile Val
               20                  25                  30
   gag cag cgc gtc tgt ggt gct gac atc atc cgc atc ccc cta gac gag         144
   Glu Gln Arg Val Cys Gly Ala Asp Ile Ile Arg Ile Pro Leu Asp Glu
           35                  40                  45
   gcc cgt ggc tgt gag agc gcc cgt atc aag agt gcc gtc ga                  185
   Ala Arg Gly Cys Glu Ser Ala Arg Ile Lys Ser Ala Val Glu
       50                  55                  60
   gtaagtccat cagccgtctt cacatgttac ggttcccgac tgacctctca tag g cac        242
                                                                His
   atg acc aac acc agc ttc gag gtc gtc gtc agc ctc ccg acg tgg aca         290
   Met Thr Asn Thr Ser Phe Glu Val Val Val Ser Leu Pro Thr Trp Thr
       65                  70                  75
   gtt gac ttc atc gct caa ggc ccc atg ccg ccc agc ccg ccc tcg gtc         338
   Val Asp Phe Ile Ala Gln Gly Pro Met Pro Pro Ser Pro Pro Ser Val
   80                  85                  90                  95
   aag gtg cct cgc cca aag aac gcc ttt gtg atc tac cgc ctt gcc aac         386
   Lys Val Pro Arg Pro Lys Asn Ala Phe Val Ile Tyr Arg Leu Ala Asn
                   100                 105                 110
   cac cag cgg gtc aag gca gcg aat cct gat ctg cac aac aac gag atc t       435
   His Gln Arg Val Lys Ala Ala Asn Pro Asp Leu His Asn Asn Glu Ile
               115                 120                 125
   gtaagcattt ctcttgcccc gccgtgtcag actacaacta acacatctct cag cc        490
                                                           Ser
   aag atc atc ggc gcc atg tgg ggt cgc gag agc cca gac gtc aaa gac      538
   Lys Ile Ile Gly Ala Met Trp Gly Arg Glu Ser Pro Asp Val Lys Asp
       130                 135                 140
   gac ttc aga gac caa gct gag gaa gag aag cgt ctt cac gct ctc gct      586
   Asp Phe Arg Asp Gln Ala Glu Glu Glu Lys Arg Leu His Ala Leu Ala
   145                 150                 155                 160
   cac cca ggc tac cag tat cag cca cgc aag cct tct gag aag aag aag      634
   His Pro Gly Tyr Gln Tyr Gln Pro Arg Lys Pro Ser Glu Lys Lys Lys
                   165                 170                 175
   cgc atg acc aag aac aag ctg gcc aag atc gca gcg aga gct cgc agc      682
   Arg Met Thr Lys Asn Lys Leu Ala Lys Ile Ala Ala Arg Ala Arg Ser
               180                 185                 190
   gcc ggc gat gac atg gat gcg acc acc cca cca ccc cag gtc gct cac      730
   Ala Gly Asp Asp Met Asp Ala Thr Thr Pro Pro Pro Gln Val Ala His
           195                 200                 205
   ccg ctc gtc aca ttc aac gac gac gct cca gac cgt gtg cct ttc aac      778
   Pro Leu Val Thr Phe Asn Asp Asp Ala Pro Asp Arg Val Pro Phe Asn
       210                 215                 220
   ttc tct gct gcc ctc gcc cca ccg cag tac gcc gct cct cag gtc gat      826
   Phe Ser Ala Ala Leu Ala Pro Pro Gln Tyr Ala Ala Pro Gln Val Asp
   225                 230                 235                 240
   gcc tac gtc cca gat gag tat gcc gag cag att cgc cag gcc gag ctc      874
   Ala Tyr Val Pro Asp Glu Tyr Ala Glu Gln Ile Arg Gln Ala Glu Leu
                   245                 250                 255
   gac aac acc aac ttc gac cgt gcc gaa gct gcc ctc gac gct gac ttc      922
   Asp Asn Thr Asn Phe Asp Arg Ala Glu Ala Ala Leu Asp Ala Asp Phe
               260                 265                 270
   agc gca ttc gat gct gcc atc cac aac gtc gct ggc aat gtc gac aac      970
   Ser Ala Phe Asp Ala Ala Ile His Asn Val Ala Gly Asn Val Asp Asn
           275                 280                 285
   cca gtc gaa tct ttc gac gcc aac agc ttc aac gcc ggc gac ttc tac      1018
   Pro Val Glu Ser Phe Asp Ala Asn Ser Phe Asn Ala Gly Asp Phe Tyr
       290                 295                 300
   gac ccc tcc ttc aac gtc gac acg ttc ttc gac ttc acc gcc gcc agc      1066
   Asp Pro Ser Phe Asn Val Asp Thr Phe Phe Asp Phe Thr Ala Ala Ser
   305                 310                 315                 320
   ggc ttc gtc tac aac ggt taa                                          1087
   Gly Phe Val Tyr Asn Gly
                   325
<210> 6
   <211> 326
   <212> PRT
   <213> Mycosphaerella polygoni-cuspidati
<400> 6
   Met Thr Thr Ser Ile Thr Thr His Pro Pro Gln Ala Pro Leu Pro Glu
   1               5                   10                  15
   Leu Asp Gln Phe Phe Leu Asn Glu Thr Arg Ile Thr Asp Tyr Ile Val
               20                  25                  30
   Glu Gln Arg Val Cys Gly Ala Asp Ile Ile Arg Ile Pro Leu Asp Glu
           35                  40                  45
   Ala Arg Gly Cys Glu Ser Ala Arg Ile Lys Ser Ala Val Glu His Met
       50                  55                  60
   Thr Asn Thr Ser Phe Glu Val Val Val Ser Leu Pro Thr Trp Thr Val
   65                  70                  75                  80
   Asp Phe Ile Ala Gln Gly Pro Met Pro Pro Ser Pro Pro Ser Val Lys
                   85                  90                  95
   Val Pro Arg Pro Lys Asn Ala Phe Val Ile Tyr Arg Leu Ala Asn His
               100                 105                 110
   Gln Arg Val Lys Ala Ala Asn Pro Asp Leu His Asn Asn Glu Ile Ser
           115                 120                 125
   Lys Ile Ile Gly Ala Met Trp Gly Arg Glu Ser Pro Asp Val Lys Asp
       130                 135                 140
   Asp Phe Arg Asp Gln Ala Glu Glu Glu Lys Arg Leu His Ala Leu Ala
   145                 150                 155                 160
   His Pro Gly Tyr Gln Tyr Gln Pro Arg Lys Pro Ser Glu Lys Lys Lys
                   165                 170                 175
   Arg Met Thr Lys Asn Lys Leu Ala Lys Ile Ala Ala Arg Ala Arg Ser
               180                 185                 190
   Ala Gly Asp Asp Met Asp Ala Thr Thr Pro Pro Pro Gln Val Ala His
           195                 200                 205
   Pro Leu Val Thr Phe Asn Asp Asp Ala Pro Asp Arg Val Pro Phe Asn
       210                 215                 220
   Phe Ser Ala Ala Leu Ala Pro Pro Gln Tyr Ala Ala Pro Gln Val Asp
   225                 230                 235                 240
   Ala Tyr Val Pro Asp Glu Tyr Ala Glu Gln Ile Arg Gln Ala Glu Leu
                   245                 250                 255
   Asp Asn Thr Asn Phe Asp Arg Ala Glu Ala Ala Leu Asp Ala Asp Phe
               260                 265                 270
   Ser Ala Phe Asp Ala Ala Ile His Asn Val Ala Gly Asn Val Asp Asn
           275                 280                 285
   Pro Val Glu Ser Phe Asp Ala Asn Ser Phe Asn Ala Gly Asp Phe Tyr
       290                 295                 300
   Asp Pro Ser Phe Asn Val Asp Thr Phe Phe Asp Phe Thr Ala Ala Ser
   305                 310                 315                 320
   Gly Phe Val Tyr Asn Gly
                   325
<210> 7
   <211> 231
   <212> DNA
   <213> Mycosphaerella polygoni-cuspidati
<220>
   <221> CDS
   <222> (1)..(231)
<400> 7
   aag gtg cct cgc cca aag aac gcc ttt gtg atc tac cgc ctt gcc aac         48
   Lys Val Pro Arg Pro Lys Asn Ala Phe Val Ile Tyr Arg Leu Ala Asn
   1               5                   10                  15
   cac cag cgg gtc aag gca gcg aat cct gat ctg cac aac aac gag atc         96
   His Gln Arg Val Lys Ala Ala Asn Pro Asp Leu His Asn Asn Glu Ile
               20                  25                  30
   tcc aag atc atc ggc gcc atg tgg ggt cgc gag agc cca gac gtc aaa        144
   Ser Lys Ile Ile Gly Ala Met Trp Gly Arg Glu Ser Pro Asp Val Lys
           35                  40                  45
   gac gac ttc aga gac caa gct gag gaa gag aag cgt ctt cac gct ctc        192
   Asp Asp Phe Arg Asp Gln Ala Glu Glu Glu Lys Arg Leu His Ala Leu
       50                  55                  60
   gct cac cca ggc tac cag tat cag cca cgc aag cct tct                    231
   Ala His Pro Gly Tyr Gln Tyr Gln Pro Arg Lys Pro Ser
   65                  70                  75
<210> 8
   <211> 77
   <212> PRT
   <213> Mycosphaerella polygoni-cuspidati
<400> 8
   Lys Val Pro Arg Pro Lys Asn Ala Phe Val Ile Tyr Arg Leu Ala Asn
   1               5                   10                  15
   His Gln Arg Val Lys Ala Ala Asn Pro Asp Leu His Asn Asn Glu Ile
               20                  25                  30
   Ser Lys Ile Ile Gly Ala Met Trp Gly Arg Glu Ser Pro Asp Val Lys
           35                  40                  45
   Asp Asp Phe Arg Asp Gln Ala Glu Glu Glu Lys Arg Leu His Ala Leu
       50                  55                  60
   Ala His Pro Gly Tyr Gln Tyr Gln Pro Arg Lys Pro Ser
   65                  70                  75
<210> 9
   <211> 20
   <212> DNA
   <213> Artificial Sequence
<220>
   <223> Mating type 1-1 forward primer
<400> 9
   gttacccagc tcgtttctga 20
<210> 10
   <211> 20
   <212> DNA
   <213> Artificial Sequence
<220>
   <223> Mating type 1-1 reverse primer
<400> 10
   agtgatctgc ggcatcttct 20
<210> 11
   <211> 20
   <212> DNA
   <213> Artificial Sequence
<220>
   <223> Mating type 1-2 forward primer
<400> 11
   cgcctttgtg atctaccgcc 20
<210> 12
   <211> 20
   <212> DNA
   <213> Artificial Sequence
<220>
   <223> Mating type 1-2 reverse primer
<400> 12
   gccagcttgt tcttggtcat 20
<210> 13
   <211> 297
   <212> DNA
   <213> Mycosphaerella polygoni-cuspidati
<400> 13
   aaagagaccg ctcaactcct ggatggcatt ccgcaagttc tacaacggcg cgctctcgcc      60
   gcacactcag aaagtgatct ccaaagccct gacgaagttt tggcgtgagg accccttcgg     120
   cgccaagtgg gtcattctcg ccaaggcata cagtgtcgtg cgcggaagcc gcgagaagca     180
   agaggctccg cttgacgagt tcttcatcat ctgcgcacca ctcatcggcg tcattccgcc     240
   aaacgagtac atgcagcaga tgggatggca tctcggcccg ccaatggagg gcgacaa        297
<210> 14
   <211> 422
   <212> DNA
   <213> Mycosphaerella polygoni-cuspidati
<220>
   <221> CDS
   <222> (1)..(420)
<400> 14
   cca aag aga ccg ctc aac tcc tgg atg gca ttc cgc aag ttc tac aac        48
   Pro Lys Arg Pro Leu Asn Ser Trp Met Ala Phe Arg Lys Phe Tyr Asn
   1               5                   10                  15
   ggc gcg ctc tcg ccg cac act cag aaa gtg atc tcc aaa gcc ctg acg        96
   Gly Ala Leu Ser Pro His Thr Gln Lys Val Ile Ser Lys Ala Leu Thr
               20                  25                  30
   aag ttt tgg cgt gag gac ccc ttc ggc gcc aag tgg gtc att ctc gcc        144
   Lys Phe Trp Arg Glu Asp Pro Phe Gly Ala Lys Trp Val Ile Leu Ala
           35                  40                  45
   aag gca tac agt gtc gtg cgc gga agc cgc gag aag caa gag gct ccg        192
   Lys Ala Tyr Ser Val Val Arg Gly Ser Arg Glu Lys Gln Glu Ala Pro
       50                  55                  60
   ctt gac gag ttc ttc atc atc tgc gca cca ctc atc ggc gtc att ccg        240
   Leu Asp Glu Phe Phe Ile Ile Cys Ala Pro Leu Ile Gly Val Ile Pro
   65                  70                  75                  80
   cca aac gag tac atg cag cag atg gga tgg cat ctc ggc ccg cca atg        288
   Pro Asn Glu Tyr Met Gln Gln Met Gly Trp His Leu Gly Pro Pro Met
                   85                  90                  95
   gag ggc gac aag gag aag atg ccg cag atc act cgc ctc ttc atc cca        336
   Glu Gly Asp Lys Glu Lys Met Pro Gln Ile Thr Arg Leu Phe Ile Pro
               100                 105                 110
   aac ctc gac acc ttt cct gat gcg atc acg atg acc aca ctc agt gtc        384
   Asn Leu Asp Thr Phe Pro Asp Ala Ile Thr Met Thr Thr Leu Ser Val
           115                 120                 125
   gac gat ctg gtc cag gcc tgc tac aac gcc ggc tac gt                     422
   Asp Asp Leu Val Gln Ala Cys Tyr Asn Ala Gly Tyr
       130                 135                 140
<210> 15
   <211> 140
   <212> PRT
   <213> Mycosphaerella polygoni-cuspidati
<400> 15
   Pro Lys Arg Pro Leu Asn Ser Trp Met Ala Phe Arg Lys Phe Tyr Asn
   1               5                   10                  15
   Gly Ala Leu Ser Pro His Thr Gln Lys Val Ile Ser Lys Ala Leu Thr
               20                  25                  30
   Lys Phe Trp Arg Glu Asp Pro Phe Gly Ala Lys Trp Val Ile Leu Ala
           35                  40                  45
   Lys Ala Tyr Ser Val Val Arg Gly Ser Arg Glu Lys Gln Glu Ala Pro
       50                  55                  60
   Leu Asp Glu Phe Phe Ile Ile Cys Ala Pro Leu Ile Gly Val Ile Pro
   65                  70                  75                  80
   Pro Asn Glu Tyr Met Gln Gln Met Gly Trp His Leu Gly Pro Pro Met
                   85                  90                  95
   Glu Gly Asp Lys Glu Lys Met Pro Gln Ile Thr Arg Leu Phe Ile Pro
               100                 105                 110
   Asn Leu Asp Thr Phe Pro Asp Ala Ile Thr Met Thr Thr Leu Ser Val
           115                 120                 125
   Asp Asp Leu Val Gln Ala Cys Tyr Asn Ala Gly Tyr
       130                 135                 140
<210> 16
   <211> 231
   <212> DNA
   <213> Mycosphaerella polygoni-cuspidati
<400> 16
   ggtgcctcgc ccaaagaacg cctttgtgat ctaccgcctt gccaaccacc agcgggtcaa      60
   ggcagcgaat cctgatctgc acaacaacga gatctccaag atcatcggcg ccatgtgggg     120
   tcgcgagagc ccagacgtca aagacgactt cagagaccaa gctgaggaag agaagcgtct     180
   tcacgctctc gctcacccag gctaccagta tcagccacgc aagccttctg a              231
<210> 17
   <211> 222
   <212> DNA
   <213> Mycosphaerella polygoni-cuspidati
<220>
   <221> CDS
   <222> (1)..(222)
<400> 17
   cgc cca aag aac gcc ttt gtg atc tac cgc ctt gcc aac cac cag cgg        48
   Arg Pro Lys Asn Ala Phe Val Ile Tyr Arg Leu Ala Asn His Gln Arg
   1               5                   10                  15
   gtc aag gca gcg aat cct gat ctg cac aac aac gag atc tcc aag atc        96
   Val Lys Ala Ala Asn Pro Asp Leu His Asn Asn Glu Ile Ser Lys Ile
               20                  25                  30
   atc ggc gcc atg tgg ggt cgc gag agc cca gac gtc aaa gac gac ttc       144
   Ile Gly Ala Met Trp Gly Arg Glu Ser Pro Asp Val Lys Asp Asp Phe
           35                  40                  45
   aga gac caa gct gag gaa gag aag cgt ctt cac gct ctc gct cac cca       192
   Arg Asp Gln Ala Glu Glu Glu Lys Arg Leu His Ala Leu Ala His Pro
       50                  55                  60
   ggc tac cag tat cag cca cgc aag cct tct                               222
   Gly Tyr Gln Tyr Gln Pro Arg Lys Pro Ser
   65                  70
<210> 18
   <211> 74
   <212> PRT
   <213> Mycosphaerella polygoni-cuspidati
<400> 18
   Arg Pro Lys Asn Ala Phe Val Ile Tyr Arg Leu Ala Asn His Gln Arg
   1               5                   10                  15
   Val Lys Ala Ala Asn Pro Asp Leu His Asn Asn Glu Ile Ser Lys Ile
               20                  25                  30
   Ile Gly Ala Met Trp Gly Arg Glu Ser Pro Asp Val Lys Asp Asp Phe
           35                  40                  45
   Arg Asp Gln Ala Glu Glu Glu Lys Arg Leu His Ala Leu Ala His Pro
       50                  55                  60
   Gly Tyr Gln Tyr Gln Pro Arg Lys Pro Ser
   65                  70

## Description

### Field of the Invention

The present invention relates to the use of a consortium of cells of the species *Mycosphaerella polygoni-cuspidati* for the herbicidal control of a plant. The invention also relates to a method for herbicidal control of a plant of the genus *Fallopia.* In addition, the disclosure herein relates to a method of producing a consortium of cells of the species *Mycosphaerella polygoni-cuspidati.* The invention further relates to a composition for the herbicidal control of a plant of the genus *Fallopia.*

### Background of the Invention

Introduced in the 19^{th} century for ornamental purposes, Japanese knotweed (*Fallopia japonica*) has spread dramatically throughout the UK and continental Europe, as well as many parts of the US and Canada. Today, the species is listed as one of the world's top 100 invaders.

In contrast to its native Japanese range, in its exotic ranges, Japanese knotweed grows aggressively, forming dense stands with severe impacts on native biodiversity. Rapid rhizomatous growth enables Japanese knotweed to force its way through concrete and tarmac with serious consequences to local infrastructures. The estimated annual cost to the British economy is currently £166 million (Williams et al. (2010) The Economic Cost of Invasive Non-Native Species on Great Britain; CABI E-UK report). Mechanical and/or chemical control of Japanese knotweed is both costly and labour-intensive. Thus, classical biological control of Japanese knotweed i.e. the introduction to its exotic ranges of co-evolved, host-specific natural enemies from its native range, which form self-sustaining populations, is an ideal additional tool for the integrated management of Japanese knotweed.

In addition, to Japanese knotweed (*Fallopia japonica*), the closely related species giant knotweed (*Fallopia sachalinensis*) and a hybrid between these two species, *Fallopia* x *bohemica,* also pose threats as invasive species.

The ascomycete fungus *Mycosphaerella polygoni-cuspidati* is a damaging, co-evolved natural enemy of Japanese knotweed which is found ubiquitously in Japan. Infection with the pathogen causes severe necrotic lesions on Japanese knotweed leaves leading to premature senescence and ultimately leaf drop. It was therefore believed that *M. polygoni-cuspidati* was a highly promising subject for classical biological control of Japanese knotweed (see for example CABI Annual Report, Europe UK, 2013; Progress with Weed Biocontrol projects, CABI in the UK, June 2013). However, the problem with this proposal is that testing against a list of 91 native species revealed that *M. polygoni-cuspidati* causes non-target effects on certain other plant species. More specifically, *M. polygoni-cuspidati* causes non-target effects on the rare British native plant species *Polygonum maritimum* and also, to a lesser degree, the species *Persicaria hydropiper.* In particular, *M. polygoni-cuspidati* infection results in necrotic lesions and the formation of spermogonia on *P*. *maritimum,* which constitute the first stage of the ascomycete life cycle. Due to the potential risk posed to *P. maritimum,* the introduction of *M. polygoni-cuspidati* in the UK as part of a classical biological control programme against Japanese knotweed would be ill-advised in case there was inadvertent infection of, and thus risk to, *P. maritimum* or other native flora. It is also possible, and perhaps likely, that other countries in the exotic range of Japanese knotweed also have native species that would be at risk from *M. polygoni-cuspidati.* As a consequence, the use of *M. polygoni-cuspidati* as a classical control agent with a self-sustaining and augmenting field population was seen to be unviable.

The present invention arises out of the recognition that *P. maritimum* generally occupies a different habitat (i.e. a coastal habitat) compared with Japanese knotweed and that, therefore, if it were possible to limit the spread of *M. polygoni-cuspidati* then the fungus could be applied to Japanese knotweed as a mycoherbicide, without risking *P. maritimum.* The invention further arises from the recognition that *M*. *polygoni-cuspidati* is heterothallic (that is to say, mating between distinct isolates possessing complementary mating genes, MAT-1 and MAT-2, is required for the development of ascospores and for completion of its life cycle) and it has no asexual conidial stage. This unusual combination of characteristics has resulted in the surprising realization that if *M. polygoni-cuspidati* were applied in the form of only a single mating type then the fungus would not develop ascospores, and, as the fungus does not produce conidia either, this prevents the fungus from reproducing and spreading in the field beyond the site of application. The spread of *M. polygoni-cuspidati* can therefore be very closely controlled. Thus applying *M. polygoni-cuspidati* in the form of only a single mating type enables *M. polygoni-cuspidati* to be used as a herbicide against Japanese knotweed.

### Summary of the Invention

According to a first aspect of the present invention, there is provided the use of a consortium of cells of the species *Mycosphaerella polygoni-cuspidati* for the herbicidal control of a plant of the genus *Fallopia,* wherein the cells in the consortium are all of the same mating type.

Conveniently, the plant is of the species or hybrid: *Fallopia japonica, Fallopia sachalinensis* or *Fallopia* x *bohemica* preferably *Fallopia japonica* var. *japonica.*

Preferably, the cells are mycelium cells.

Advantageously, the *Mycosphaerella polygoni-cuspidati* cells in the consortium are all of mating type 1.

Alternatively, the *Mycosphaerella polygoni-cuspidati* cells in the consortium are all of mating type 2.

According to a second aspect of the present invention, there is provided a method for herbicidal control of a plant of the genus *Fallopia* comprising contacting the plant with a composition comprising a consortium of cells of the species *Mycosphaerella polygoni-cuspidati,* wherein the cells in the consortium are all of the same mating type.

Conveniently, the method further comprises the step of maintaining the cells in an aqueous environment on the plant for at least 12 hours, preferably at least 24 hours.

Preferably, the method comprises contacting the leaves of the plant with the composition.

Advantageously, the plant is of the species or hybrid: *Fallopia japonica, Fallopia sachalinensis* or *Fallopia* x *bohemica* preferably *Fallopia japonica* var. *japonica.*

Coveniently, the cells are mycelium cells.

Preferably, the *Mycosphaerella polygoni-cuspidati* cells in the consortium are all of mating type 1.

Alternatively, the *Mycosphaerella polygoni-cuspidati* cells in the consortium are all of mating type 2.

Disclosed herein, there is provided a method of producing a consortium of cells of the species *Mycosphaerella polygoni-cuspidati* wherein the cells in the consortium are all of the same mating type comprising the steps of:
i. isolating a first individual ascospore from a sample of ascospores of the species Mycosphaerella polygoni-cuspidati; and
ii. generating at least a first mycelium from said first isolated ascospore.

Conveniently, the disclosed method further comprises the step of:
iii. determining the mating type of said first mycelium.

Preferably, the disclosed method further comprises the steps of:
iv. isolating a second individual ascospore from a sample of ascospores of the species Mycosphaerella polygoni-cuspidati;
v. generating at least a second mycelium from said second isolated ascospore;
vi. determining the mating type of said second mycelium;
vii. confirming that the mating type of the first mycelium is the same as the mating type of the second mycelium;
viii. mixing the first and second mycelia.

Advantageously, step iii and/or step vi of the disclosed method comprise detecting the presence of a biological marker specific for one mating type.

Conveniently, the biological marker is a polynucleotide of the sequence of SEQ. ID NO: 1 or 5 or a fragment thereof which is at least 20, preferably at least 50 nucleotides in length.

According to a third aspect of the present invention, there is provided a composition for the herbicidal control of a plant of the genus *Fallopia* comprising a consortium of cells of the species *Mycosphaerella polygoni-cuspidati* or the like, wherein the cells in the consortium are all of the same mating type, further comprising an adhesive agent and/or a wetting agent.

Conveniently, the cells are mycelium cells.

Preferably, the *Mycosphaerella polygoni-cuspidati* cells in the consortium are all of mating type 1.

Alternatively, the *Mycosphaerella polygoni-cuspidati* cells in the consortium are all of mating type 2.

Disclosed herein, there is provided a polynucleotide primer comprising the sequence of any one of SEQ. ID NOS: 9 to 12.

Disclosed herein, there is provided a method for herbicidal control of a plant comprising contacting the plant with a composition comprising a consortium of cells of a fungal species which has the following characteristics:
i. the fungal species is capable of infecting the plant and causing necrosis in the plant;
ii. the fungal species has no asexual stage in its life cycle;
iii. the fungal species is heterothallic,
wherein the cells of the fungal species in the consortium are all of the same mating type.

### Definitions

As used herein, the term *"Mycosphaerella polygoni-cuspidati"* or *"M*. *polygoni-cuspidati"* means a fungus in accordance with the taxonomy set forth in Kurose et al., (2009) Systematics of Mycosphaerella species associated with the invasive weed Fallopia japonica, including the potential biological control agent M. polygoni-cuspidati. Mycoscience 50, 179-189.

As used herein, the term *"Mycosphaerella polygoni-cuspidati* or the like" means the fungus *Mycosphaerella polygoni-cuspidati* and species having at least 98% similarity (or 98% sequence identity) in their ITS (internal transcribed spacer) region to the ITS of *Mycosphaerella polygoni-cuspidati* and meeting the following requirements: i) the fungus infects, and has a herbicidal effect on, a plant of the genus *Fallopia*; ii) the fungus cycles exclusively through its sexual stage (and has no asexual reproduction) and iii) the fungus is heterothallic.

As used herein, the term "herbicidal control" means reduction or elimination of plant health or growth and includes (but is not limited to) exfoliation and plant death.

As used herein, the term "a plant of the genus *Fallopia*" means any plant of the genus *Fallopia.* In some embodiments it is defined as any invasive plant of the genus *Fallopia.* It includes the species *Fallopia japonica* (which is also known as Japanese Knotweed) and *Fallopia sachalinensis* and the hybrid thereof, *Fallopia* x *bohemica.* It also includes other hybrid species of the aforementioned species, in particular other hybrids of *Fallopia japonica.*

As used herein, the term "consortium of cells" means a collection or mixture of cells. In some embodiments the term may be replaced with the term "aggregate of cells" throughout the specification.

As used herein, the term "mating type" means the characteristics that define two sexually compatible individuals.

The percentage "identity" between two sequences may be determined using the BLASTP algorithm version 2.2.2 (Altschul, Stephen F., Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402) using default parameters. In particular, the BLAST algorithm can be accessed on the internet using the URL http://www.ncbi.nlm.nih.gov/blast/.

### Figures

Figure 1 is a schematic diagram of the life cycle of *M*. *polygoni-cuspidati.*
Figure 2 shows photographs of disease symptoms of *M*. *polygoni-cuspidati* (ex Mt Hiko, Japan) on Japanese knotweed at four points in the life cycle of *M*. *polygoni-cuspidati.*
Figure 3 is a diagram of the life cycle of *M. polygoni-cuspidati.*
Figure 4 is a photograph of disease symptoms of *M. polygoni-cuspidati* (ex Mt Hiko, Japan) on Japanese knotweed 2.5 months after inoculation with ascospores in quarantine greenhouse.
Figure 5 is a photograph of disease symptoms of *M. polygoni-cuspidati* (ex Mt Hiko, Japan) on Japanese knotweed 2.5 months after inoculation with ascospores in quarantine greenhouse, top leaf with white label over it is the control leaf.
Figure 6 is a photograph of Japanese knotweed leaf material infected with *M. polygoni-cuspidati,* bearing mature pseudothecia (right hand side) and leaf discs, conditioned in sterile distilled water for ascospore drop, affixed to petri dish lids for suspension over agar to obtain ascospore drop.
Figure 7 is a photograph of PDA (potato dextrose agar) broth inoculated with mycelial plugs of *M. polygoni-cuspidati* after one week incubation on a shaker.
Figure 8 is a photograph of macerated mycelium of *M. polygoni-cuspidati* used for inoculations.
Figure 9 is a photograph of disease symptoms of *M. polygoni-cuspidati* on Japanese knotweed circa 3 weeks after inoculation with mycelium in quarantine greenhouse.
Figure 10 is a photograph of disease symptoms of *M. polygoni-cuspidati* (ex Mt Hiko, Japan) on Japanese knotweed 1 month after inoculation with mycelial broth in quarantine greenhouse.

### Detailed Description

### Mycosphaerella polygoni-cuspidati or the like

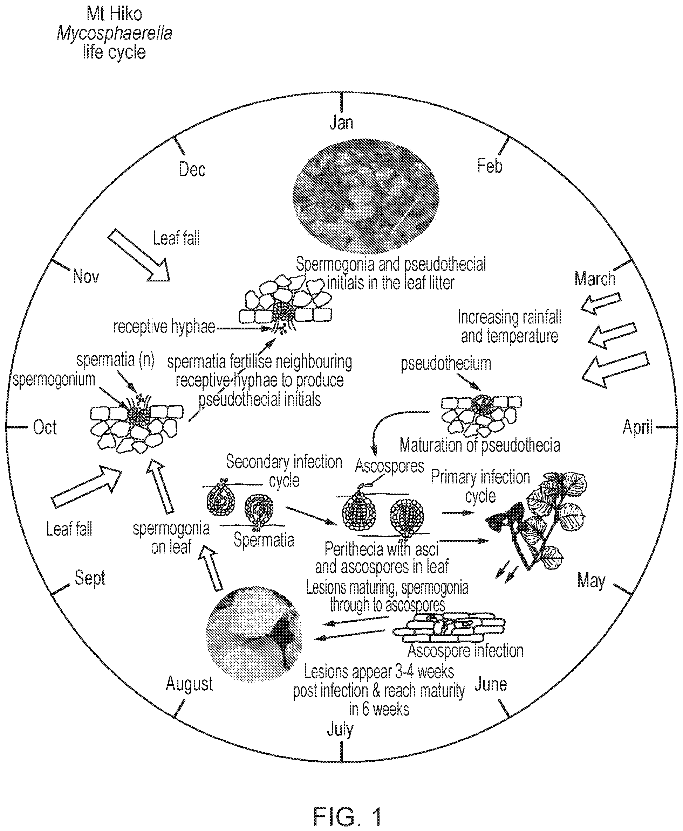

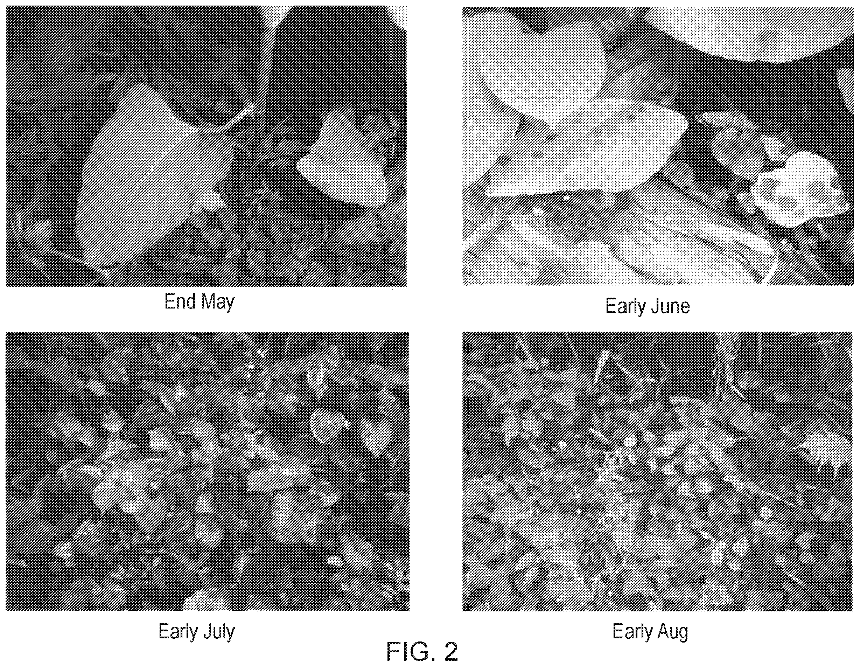

The ascomycete *Mycosphaerella polygoni-cuspidati* (Mycosphaerellaceae, scomycota), or Mycosphaerella leaf-spot, is a fungal pathogen associated with Japanese knotweed (*Fallopia japonica)* in its native range in Japan. It has been shown that the pathogen is widespread in Japan causing extensive damage to its host and to exhibit apparent field host-specificity. Figure 1 describes the life cycle of the fungus and Figure 2 shows the disease symptoms of the fungus on Japanese knotweed at the end of May, in early June, in early July and in early August. The typical symptoms of the disease caused by the pathogen are chestnut brown circular lesions on the upper leaf surface which are surrounded by a darker, water-soaked halo. With progressing infection these individual lesions coalesce. On the lower leaf surface the appearance of the lesions is pale brown with a prominent grayish-brown halo and whitened veins. Heavy infection results in extensive leaf necrosis, leaf distortion and premature defoliation. Most commonly observed in the necrotic lesions are spermogonia as the first fruiting bodies appearing in the life-cycle. Spermogonia are dark brown to black, immersed to semi-erumpent and appear sometimes in concentric rings within lesions. They occur on both leaf surfaces but are more prominent on the upper leaf surface. Ascomata or pseudothecia as the fruiting bodies appearing subsequently in the life cycle, are less frequent, they are larger and more erumpent than the spermogonia.

The ascomycete life cycle generally consists of two stages: a sexual stage during which ascospores are produced as a result of spermatial mating and recombination, as well as an asexual stage characterized by the production of high numbers of conidia for dispersal. *Mycosphaerella polygoni-cuspidati* is known to produce exclusively the sexual ascospore stage, whilst conidia are lacking from the life cycle. Molecular studies using specifically developed markers have further proved the fungus to be heterothallic meaning that mating between two different isolates with complementary spermatial sex gametes (mating type 1 and mating type 2) needs to take place in order for infective ascospores to be produced. Greenhouse inoculation studies with Japanese knotweed using one single isolate (i.e. mating type) of the pathogen have confirmed that while infection will evoke characteristic diseases symptoms including the development of spermogonia producing spermatia, the formation of ascospores is lacking and the life cycle cannot be completed.

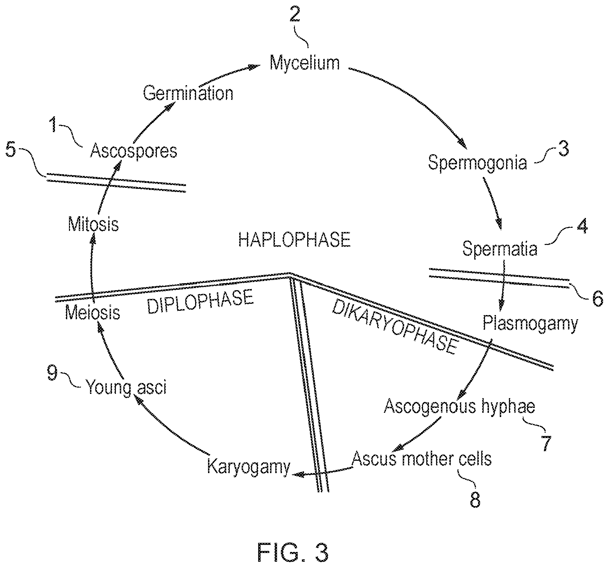

Referring to Figure 3, the life cycle pattern of *M. polygoni-cuspidati* is shown. Ascospores 1 of the fungus germinate to form mycelium 2 which infects leaves of the target (typically *Fallopia Japonica*) entering via the stomata. As a consequence of infection, circular brown lesions develop on the leaves from necrotic spots. From the lesions, spermogonia 3 grow from which spermatia 4 are produced. *M. polygoni-cuspidati* is heterothallic and the two mating types of *M. polygoni-cuspidati* are mating type 1 and mating type 2. Spermatia of mating type 1 and spermatia of mating type 2 contact with respective, receptive hyphae 7 which are thereby fertilized to produce ascus mother cells 8. The ascus mother cells 8 produce diploid young asci 9 which, through the processes of meiosis and mitosis, form the ascospores 1 at which point the cycle repeats.

It is to be noted that *M. polygoni-cuspidati* has no asexual stage to its life cycle. In particular, *M. polygoni-cuspidati* does not form conidia, which are the asexual spores of most ascomycetes. Conidia as the asexual stage of an Ascomycete have never been observed in the field, following greenhouse inoculation or in culture.

In embodiments of the present invention, cells of *M. polygoni-cuspidati* are provided. It is to be understood that the cells may be of any strain of *M. polygoni-cuspidati.* Such varieties can be characterized as having at least 98%, preferably at least 99% similiarity (identity) in the ITS region to the ITS region of *M. polygoni-cuspidati* as identified in Kurose et al and having the IMI number 401912 and the GenBank accession number AB 434912. Furthermore, the species is heterothallic, has no asexual stage and has the ability to infect F. *japonica.*

A taxonomic description of *Mycosphaerella polygoni-cuspidati* is provided in Kurose et al., (2009) Systematics of Mycosphaerella species associated with the invasive weed Fallopia japonica, including the potential biological control agent M. polygoni-cuspidati. Mycoscience 50, 179-189. The fungus was originally described by Hara, Byotyugai Zasshi 5: 617, 1918, but the specimen was lost, hence this pathogen was re-described with a neotype deposited under the IMI number 393529.

As explain above, *M. polygoni-cuspidati* has two mating types and the unique mating type sequences of *Mycosphaerella polygoni-cuspidati* are shown below.
▪ *MAT1-1-1* sequence (1,296 bp)

In a first disclosure, analysis of the cDNA *MAT1-1-1* sequence indicates that its structure is as follows.
cDNA (exon): 1-259, 309-402, 458-626, 679-1,296 bp
intron: 260-308, 403-457, 627-678 bp
α-box: 223-259, 309-402, 458-623 bp

Therefore the amino acid sequence of MAT1-1-1 encoded by the nucleotide sequence of SEQ. ID NO: 1 can be predicted as follows.

Sequence of α-box region in *MAT1-1-1* as follows.

Furthermore, the amino acid sequence of α-box in MAT1-1-1 encoded by the nucleotide sequence of SEQ. ID NO: 3 can be predicted as follows.

In a second disclosure, analysis of the cDNA *MAT1-1-1* sequence indicates that its structure is as follows.
cDNA (exon): 1-259, 309-402, 458-626, 679-1,296 bp
intron: 260-308, 403-457, 627-678 bp
α-box: 225-259, 309-402, 458-625 bp

Sequence of α-box region in MAT1-1-1 as follows.

In a third disclosure, analysis of the cDNA MAT1-1-1 sequence indicates that its structure is as follows.
cDNA (exon): 1-259, 309-402, 458-626, 679-1,296 bp
intron: 260-308, 403-457, 627-678 bp
α-box: 223-259, 309-402, 458-626, 679-800 bp

Sequence of α-box region in *MAT1-1-1* as follows.

Furthermore, the amino acid sequence of α-box in MAT1-1-1 encoded by the nucleotide sequence of SEQ. ID NO: 14 can be predicted as follows (based on Conserved Domain Database, NCBI which can be accessed on the internet using the URL http://www.ncbi.nlm.nih.gov/cdd/).
▪ *MAT1-2-1* (1,087 bp)

In a first disclosure, analysis of the cDNA *MAT1-2-1* sequence indicates that its structure is as follows.
cDNA (exon): 1-185, 239-435, 489-1,087 bp
intron: 186-238, 436-488 bp
HMG box: 339-435, 489-622 bp

Therefore the amino acid sequence of MAT1-2-1 encoded by the nucleotide sequence of SEQ. ID NO: 5 can be predicted as follows.

Sequence of HMG box region in *MAT1-2-1* as follows.

Furthermore, the amino acid sequence of HMG box in MAT1-2-1 encoded by the nucleotide sequence of SEQ. ID NO: 7 can be predicted as follows.

In a second disclosure, analysis of the cDNA *MAT1-2-1* sequence indicates that its structure is as follows.
cDNA (exon): 1-185, 239-435, 489-1,087 bp
intron: 186-238, 436-488 bp
HMG box: 341-435, 489-624 bp

Sequence of HMG box region in *MAT1-2-1* as follows.

In a third disclosure, analysis of the cDNA *MAT1-2-1* sequence indicates that its structure is as follows.
cDNA (exon): 1-185, 239-435, 489-1,087 bp
intron: 186-238, 436-488 bp
HMG box: 348-435, 489-622 bp

Sequence of HMG box region in *MAT1-2-1* as follows.

Furthermore, the amino acid sequence of α-box in MAT1-1-1 encoded by the nucleotide sequence of SEQ. ID NO: 17 can be predicted as follows (based on Conserved Domain Database, NCBI which can be accessed on the internet using the URL http://www.ncbi.nlm.nih.gov/cdd/).

### Risk of Crossing/Hybridization between M. polygoni-cuspidati and other Fungal Pathogens

It is considered unlikely that *M. polygoni-cuspidati* could cross or hybridize with other fungal pathogens or *Mycosphaerella* species in the UK. The taxonomic name and position of the pathogen are as follows: ***Mycosphaerella polygoni-cuspidati* (Mycosphaerellaceae, Mycosphaerellales, Dothideomycetidae, Dothideomycetes, Ascomycota, Fungi).** Morphological characteristics place the leaf-spot pathogen firmly in the genus *Mycosphaerella.* Based on morphological data, specifically ascus and ascospore dimensions, as well as molecular data, this leaf spot also falls within the species concept, as described by Hara (1918) from leaf litter from the island of Honshu. But, as no type was designated a neotype description has been published (Kurose *et al.,* 2009) Molecular analysis showed *M. polygoni-cuspidati* sequences (ITS region) to have no close match to those documented in molecular databases (i.e. GenBank). *Mycosphaerella sumatrensis,* a recently described species on *Eucalyptus* from Indonesia, showed the nearest match (96-97 % homology in ITS region), thus indicating it to be a different species. The low level of homology between *M. polygoni-cuspidati* and *M*. *sumatrensis* might indicate that the species on *Fallopia japonica* var. *japonica is* taxonomically isolated. A BLAST search gave matches with a number of other *Mycosphaerella* species and their anamorphs but none of these showed more than 90% homology (i.e. *Mycosphaerella areola* 89%, *Mycosphaerella microsora* 89%, *Mycosphaerella brassicicola* 88%).

In the field, fungal species are known to hybridize with closely related species. "The key question, whether a newly formed fungal hybrid will persist in nature, is directly related to the relative fitness of the hybrid" (Schardl & Craven, 2003, Interspecific hybridization in plant-associated fungi and oomycetes: a review. Molecular Ecology 12: 2861-2873). Hybridization resulting in more virulent hybrids has been shown to occur in the genera *Phytophthora, Ophiostoma* and *Melampsora* (Brasier, 2001, Rapid evolution of introduced plant pathogens via interspecific hybridization. BioScience 51: 123-133; Schardl & Craven, 2003). On the other hand, based on research undertaken with *Heterobasidion annosum,* Garbelotto et al. (2007) Ecological constraints limit the fitness of fungal hybrids in the Heterobasidion annosum species complex. Applied Environmental Microbiology, 73: 6106-6111 propose that "in fungal species complexes characterized by differential host specificity, hybrids may not be generally unfit but may be at an ecological disadvantage with regard to their parents in a habitat specific for one of the parents."

Thus molecular data strongly indicate that there are no closely-related *Mycosphaerella* species with which to hybridise. Furthermore, by using a single mating type isolate for application as a mycoherbicide *M. polygoni-cuspidati* will not be able to produce ascospores in the field, thus will neither reproduce nor spread beyond the area of application. Given that no other *Mycosphaerella* species is known to attack Japanese knotweed outside its native range Japan and *M. polygoni-cuspidati* will only reside in treated Japanese knotweed plants, hybridization will not take place.

### Genus Fallopia

The genus *Fallopia* comprises around a dozen species of flowering plants in the family Polygonaceae. In embodiments of the present invention, invasive plants of the genus are preferred and plants of the species *Fallopia japonica* (which is commonly known as "Japanese knotweed") are particularly preferred. Plants of the variety *Fallopia japonica* var. *japonica* are more particularly preferred. However, other embodiments of the invention relate to plants of the species *Fallopia sachalinensis* (which is commonly known as "giant knotweed"). Still further embodiments relate to plants of the hybrid *Fallopia* x *bohemica* which is a hybrid of the species *Fallopia japonica* and *Fallopia sachalinensis.* It is to be understood that the invention also relates to other species within the genus *Fallopia* such as hybrids of *Fallopia japonica* with species other than *Fallopia sachalinensis.*

### Herbicidal Product

The principal component of the herbicidal product of the invention is a consortium of cells. In one embodiment of the present invention, the herbicidal products comprises a consortium of cells of *M. polygoni-cuspidati.* In some embodiments, the consortium comprises only cells of a single species but in other embodiments, the consortium comprises cells of a plurality of species such as, for example, i) cells of a first strain of *M. polygoni-cuspidati,* ii) cells of a second strain of *M. polygoni-cuspidati* and iii) cells of a different species. It is to be understood that all of the cells of *M. polygoni-cuspidati* and any other cells which are capable of mating with *M. polygoni-cuspidati* are of the same mating type (i.e. either all are mating type 1 or all are mating type 2). However, other cells, which do not mate with *M. polygoni-cuspidati,* may, if they have a mating type, be of any mating type.

It is preferred that the cells in the consortium are mycelium cells. It is to be understood that these are the haploid mycelial cells that arise from ascospores' germination from and not mycelia from the dikaryophase of the life cycle. In one embodiment, the cells in the consortium are individual, separate cells but in other embodiments, the cells are in the form of part or all of a mycelium or mycelia. In further embodiments, the cells of the consortium are ascospores and in still further embodiments the cells are a mixture of mycelium cells and ascospores.

As disclosed herein, the consortium of cells is provided as follows. Infected plants are readily available in Japanese knotweed stands at Mount Hiko. Collections of the pathogen are made by sampling infected leaves displaying the above described disease symptoms. Leaves are dried in a plant press for 3-5 days with a daily change of newspaper as the drying paper and then transferred into wax packets.

Using a stereomicroscope, the leaf spots are assessed for mature pseudothecia of *M. polygoni-cuspidati.* Leaf discs containing these pseudothecia are cut using a hole-punch and immersed in sterile distilled water at 19°C for 24 hours. Leaf discs (pseudothecia facing down) are affixed to the lid of a Petri dish using double sided sticky tape, suspended over tap water agar (TWA) and incubated at 19°C for 24 hours. After this incubation period plates are microscopically examined for ascospore discharge. *Mycosphaerella polygoni-cuspidati* ascopores are identified based on morphology and single spore isolates are made by removing individual ascospores together with a block of agar using a sterile hypodermic needle. Each block is placed onto separate TWA agar plates and placed in an incubator at 19°C. Each single spore isolate is then molecularly characterized with respect to its mating type.

Multiplex PCR is performed on samples of each spore isolate. Primer sets for differentiating the two mating types are shown below. These primers have been designed originally based on the sequence of each mating type shown above.
Primer sets for detecting Mating type 1-1 isolates
   Forward primer: GTTACCCAGCTCGTTTCTGA (SEQ. ID NO: 9)
   Reverse primer: AGTGATCTGCGGCATCTTCT (SEQ. ID NO: 10)
Primer sets for detecting Mating type 1-2 isolates
   Forward: CGCCTTTGTGATCTACCGCC (SEQ. ID NO: 11)
   Reverse: GCCAGCTTGTTCTTGGTCAT (SEQ. ID NO: 12)

However, it is to be appreciated that mating type characterization may be performed in accordance with alternative methods, for example by detecting the mating type sequences using different sets of primer sequences that are specific for each mating type isolate.

After mating type characterization, a mycelial culture is grown up which contains the consortium of cells of the invention. In some disclosed methods, multiple mycelia cultures of the same mating type are mixed together in order to provide sufficient quantities of cells.

In some embodiments, the herbicidal product also comprises such further additives and/or adjuvants, in addition to the consortium of cells, as are appropriate to provide a practical crop protection product. Additives are provided to maintain the long-term viability of the consortium's propagule and to facilitate ease of handling, storage and application. In preferred embodiments, additives are provided so that the herbicidal product is in the form of a liquid or solution. For example, in one embodiment, the mycelium grown in potato dextrose broth and thus the broth itself may be used in order that the herbicidal product is in the form of a liquid. In another embodiment, the consortium comprises an additive that enhances adherence of the consortium to plant leaves. In another embodiment, the consortium comprises an additive, such as a wetting agent, that maintains the cells in an aqueous environment for an extended period such as at least 12 hours and preferably at least 24 hours. Adjuvants are provided to improve spray delivery of the consortium or enhance the pathological impacts of the propagules. Further details of suitable additives and adjuvants are provided in *Fungi As Biocontrol Agents: Progress Problems and Potential* edited by T. M. Butt, C. Jackson, N. Magan.

In some embodiments the concentration is 2x10⁵ mycelial fragments per ml. However, in other embodiments, the concentration is between 1x10⁴ and 1x10⁶ mycelial fragments per ml, preferably between 1x10⁵ and 3x10⁵ mycelial fragments per ml

### Use of the consortium

In use, the consortium is inoculated on the leaves of a plant of the genus *Fallopia* as will now be described. In some embodiments, the herbicidal product comprising the consortium is painted on to the leaves of the plant using a brush. This provides very effective coverage of the leaves but is time consuming and is not appropriate for large scale coverage. Accordingly, in preferred embodiments, the herbicidal product is sprayed on to the leaves of the plant, which permits coverage of multiple plants substantially simultaneously.

The cells of the consortium then infect the plant via the leaf stomata and there is fungal growth within the plant. Subsequently, the fungal infection results in defoliation of the plant resulting in death of the plant or a severe setback to its growth which allows other plant species the opportunity to out-compete it during the growing season. Referring to Figure 3, it is to be appreciated that the life cycle of the fungus starts (in the production phase) at the initiation line 5 with the provision of ascospores 1. Furthermore, since only one cell mating type is present in the consortium, the life cycle does not proceed beyond the formation of spermatia 4 and thus stops at the termination line 6. This is very significant since, unlike spores, mycelia cannot be spread by wind and so plant infection is limited to contact only (i.e. the plant initially infected and any other plant where there is leaf-to-leaf contact). As such the effective area of the fungus is controlled and spread is prevented.

Since a single application of the herbicidal product may not result in plant death, in some embodiments of the invention, multiple applications of the herbicidal product are inoculated on the plant. Symptoms on the plant leaf are normally visible after 3-4 weeks and it is preferred that such symptoms are assessed before a second application of the product. Therefore, in some embodiments further applications are applied between 1 and 2 months are the preceding application. The number of applications required depends upon various factors such as the age of the plant and the climate.

While it is generally desirable to ensure that spraying covers only leaves of plants of the genus *Fallopia,* herbicidal product that does fall on other plants is not generally of concern since very few other plants are susceptible to infection by the fungus of the present invention. Furthermore, even if a non-target plant were infected by the fungus of the invention, it would at worst result in infection of plants in physical contact with the originally-infected plant since the presence of only a single mating type in the consortium means that the fungus cannot complete its life cycle to form ascospores. Indeed, spread of the fungus is limited in most circumstances to growth within the plant originally inoculated.

### EXAMPLES

### Example 1

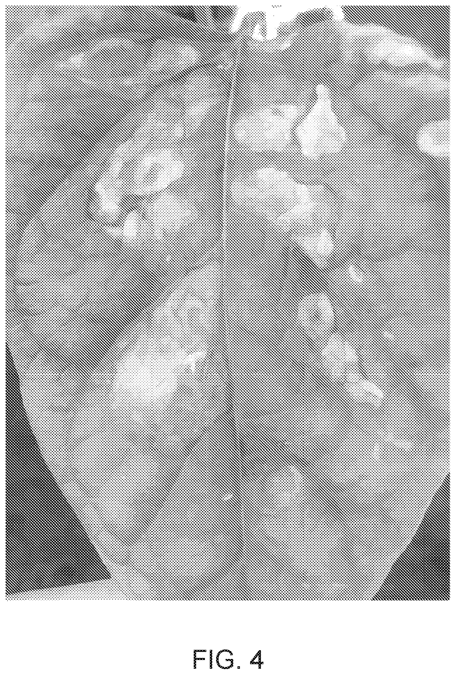

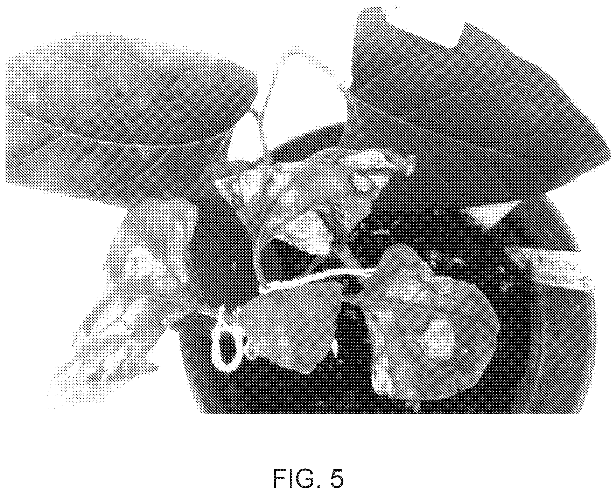

During survey work in Japan *M. polygoni-cuspidati* was commonly encountered in Japanese knotweed stands at Mount Hiko. Samples of *M. polygoni-cuspidati* were taken and ascospores thereof were inoculated onto leaves of Japanese knotweed in a quarantine greenhouse. Figure 4 shows a leaf 2.5 months after inoculation. Figure 5 shows a plant 2.5 months after inoculation in which the top leaf with a white label over it is the control leaf.

### Example 2

During survey work in Japan *M. polygoni-cuspidati* was commonly encountered in Japanese knotweed stands at Mount Hiko. Collections of the pathogen were made by sampling infected leaves displaying the above described disease symptoms. Leaves were dried in a plant press for 3-5 days with a daily change of newspaper as the drying paper and then transferred into wax packets. Samples were taken back into quarantine in the UK under licences.

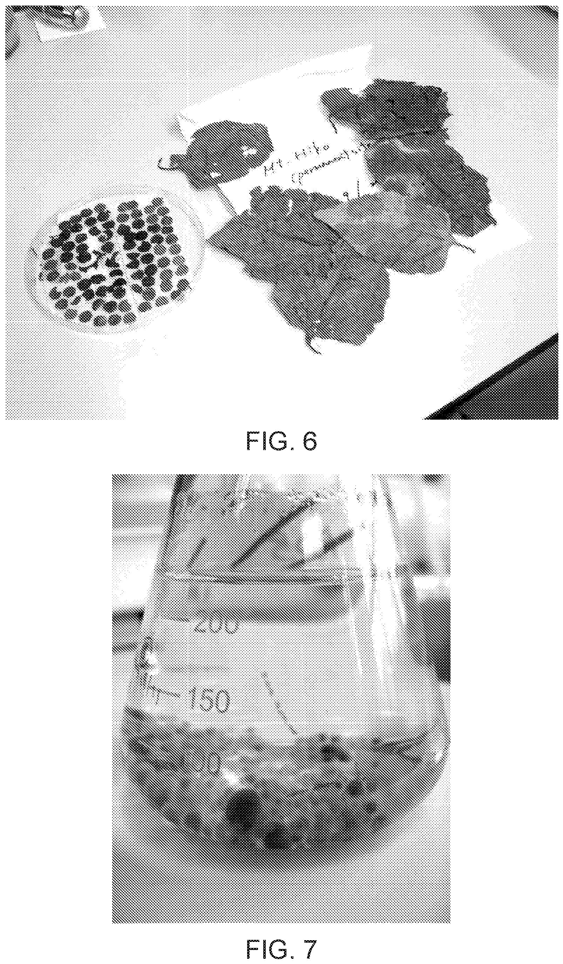

Using a stereomicroscope, the leaf spots were assessed for mature pseudothecia of *M. polygoni-cuspidati.* Leaf discs containing these pseudothecia were cut using a hole-punch and immersed in sterile distilled water at 19°C for 24 hours. Leaf discs (pseudothecia facing down) were affixed to the lid of a Petri dish using double sided sticky tape (see Figure 6), suspended over tap water agar (TWA) and incubated at 19°C for 24 hours. After this incubation period plates were microscopically examined for ascospore discharge. *Mycosphaerella polygoni-cuspidati* ascopores were identified based on morphology and single spore isolates were made by removing individual ascospores together with a block of agar using a sterile hypodermic needle. Each block was placed onto separate TWA agar plates and placed in an incubator at 19°C. Each single spore isolate can then be molecularly characterized with respect to its mating type.

### Example 3

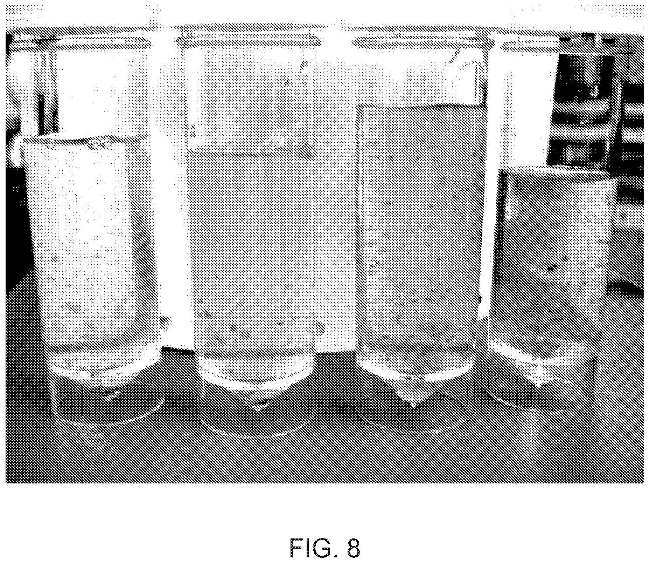

Four mycelial plugs, 5 mm in diameter, were taken from the actively growing margin of an individual culture of *M. polygoni-cuspidati* using a sterile cork-borer. The mycelial plugs were split into smaller fragments and added to 75 ml of potato dextrose broth (PDB) in a 250 ml conical flask. A flask containing PDB only was used as a control to detect potential contamination during the mass production process. Flasks were sealed with a sterile foam bung and placed on a rotary shaker at 170 rpm at 18°C for 8 days (see Figure 7). Subsequently the mycelial broth was transferred to a sterilised blender and macerated for 20 seconds in order to produce a more homogenous inoculum (Figure 8).

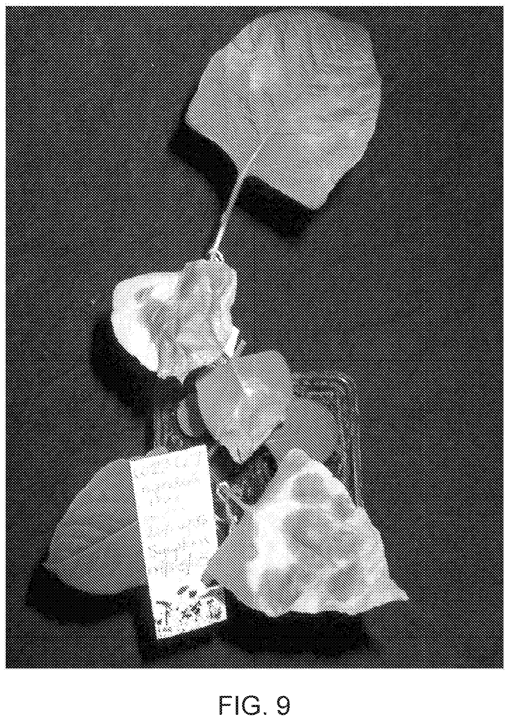

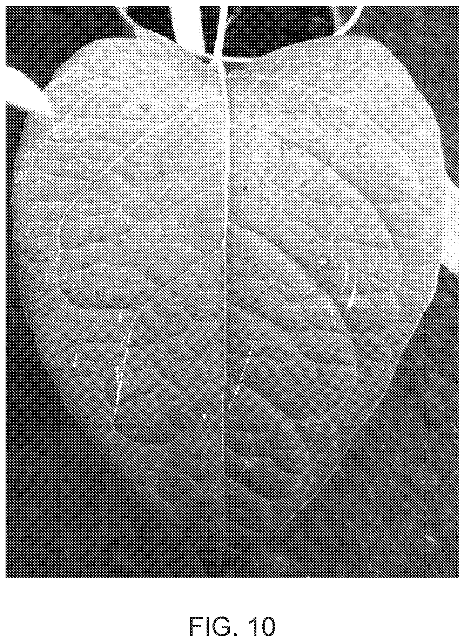

The fragmented mycelium broth was applied to plants by using a paintbrush and brushing the inoculum onto the upper and lower leaf surface. Control plants/leaves were inoculated with PDB liquid medium only. Inoculated plants were placed in a customized dew chamber (Mercia Scientific, Birmingham, UK) set at 20°C, without light for 48 h. The viability of the inoculum was assessed as growth of mycelial fragments on TWA. Upon removal from the dew chamber all plants were maintained in a designated quarantine greenhouse chamber under a controlled temperature and light regime of 20°C and 12-h dark/12-h light and regularly monitored for the development of disease symptoms. Figure 9 is a photograph of a leaf c. 3 weeks after inoculation with mycelial broth. Figure 10 is a photograph of a leaf 1 month after inoculation with mycelial broth.

## Claims

1. Use of a consortium of cells of the species *Mycosphaerella polygoni-cuspidati* for the herbicidal control of a plant of the genus *Fallopia,* wherein the cells in the consortium are all of the same mating type.

2. The use according to claim 1 wherein the plant is of the species or hybrid: *Fallopia japonica, Fallopia sachalinensis* or *Fallopia* x *bohemica,* preferably *Fallopia japonica* var. *japonica.*

3. The use according to claim 1 or 2 wherein the cells are mycelium cells.

4. The use according to any one of claims 1 to 3, wherein the *Mycosphaerella polygoni-cuspidati* cells in the consortium are all of mating type 1.

5. The use according to any one of claims 1 to 3, wherein the *Mycosphaerella polygoni-cuspidati* cells in the consortium are all of mating type 2.

6. A method for herbicidal control of a plant of the genus *Fallopia* comprising contacting the plant with a composition comprising a consortium of cells of the species *Mycosphaerella polygoni-cuspidati,* wherein the cells in the consortium are all of the same mating type.

7. The method according to claim 6, further comprising the step of maintaining the cells in an aqueous environment on the plant for at least 12 hours, preferably at least 24 hours.

8. A method according to claim 6 or 7, comprising contacting the leaves of the plant with the composition.

9. The method according to any one of claims 6 to 8, wherein the plant is of the species or hybrid: *Fallopia japonica, Fallopia sachalinensis* or *Fallopia* x *bohemica* preferably *Fallopia japonica* var. *japonica.*

10. The method according to any one of claims 6 to 9, wherein the cells are mycelium cells.

11. The method according to any one of claims 6 to 10, wherein the *Mycosphaerella polygoni-cuspidati* cells in the consortium are all of mating type 1.

12. The method according to any one of claims 6 to 10, wherein the *Mycosphaerella polygoni-cuspidati* cells in the consortium are all of mating type 2.

13. A composition for the herbicidal control of a plant of the genus *Fallopia* comprising a consortium of cells of the species *Mycosphaerella polygoni-cuspidati,* wherein the cells in the consortium are all of the same mating type, the composition further comprising an adhesive agent and/or a wetting agent.

14. A composition according to claim 13, wherein the cells are mycelium cells.

15. A composition according to claim 13 or 14, wherein the *Mycosphaerella polygoni-cuspidati* cells in the consortium are all of mating type 1 or are all of mating type 2.

## Patentansprüche

1. Verwendung eines Konsortiums von Zellen der Spezies *Mycosphaerella polygoni-cuspidati* zur herbiziden Kontrolle einer Pflanze der Gattung *Fallopia,* wobei die Zellen im Konsortium alle vom gleichen Paarungstyp sind.

2. Verwendung nach Anspruch 1, wobei die Pflanze zu der Spezies oder dem Hybrid gehört: *Fallopia japonica, Fallopia sachalinensis* oder *Fallopia x bohemica,* bevorzugt *Fallopia japonica var. japonica.*

3. Verwendung nach Anspruch 1 oder 2, wobei die Zellen Myzelzellen sind.

4. Verwendung nach einem der Ansprüche 1 bis 3, wobei die *Mycosphaerellapolygoni-cuspidati-Zellen* im Konsortium alle vom Paarungstyp 1 sind.

5. Verwendung nach einem der Ansprüche 1 bis 3, wobei die *Mycosphaerellapolygoni-cuspidati*-Zellen im Konsortium alle vom Paarungstyp 2 sind.

6. Verfahren zur herbiziden Kontrolle einer Pflanze der Gattung *Fallopia,* umfassend In-Kontakt-Bringen der Pflanze mit einer Zusammensetzung, die ein Konsortium von Zellen der Spezies *Mycosphaerella polygoni-cuspidati* umfasst, wobei die Zellen im Konsortium alle vom gleichen Paarungstyp sind.

7. Verfahren nach Anspruch 6, weiterhin umfassend den Schritt des Haltens der Zellen in einer wässrigen Umgebung auf der Pflanze für mindestens 12 Stunden, bevorzugt für mindestens 24 Stunden.

8. Verfahren nach Anspruch 6 oder 7, umfassend In-Kontakt-Bringen der Blätter der Pflanze mit der Zusammensetzung.

9. Verfahren nach einem der Ansprüche 6 bis 8, wobei die Pflanze zu der Spezies oder dem Hybrid gehört: *Fallopia japonica, Fallopia sachalinensis* oder *Fallopia x bohemica,* bevorzugt *Fallopia japonica var. japonica.*

10. Verfahren nach einem der Ansprüche 6 bis 9, wobei die Zellen Myzelzellen sind.

11. Verfahren nach einem der Ansprüche 6 bis 10, wobei die *Mycosphaerellapolygoni-cuspidati*-Zellen im Konsortium alle vom Paarungstyp 1 sind.

12. Verfahren nach einem der Ansprüche 6 bis 10, wobei die *Mycosphaerellapolygoni-cuspidati-Zellen* im Konsortium alle vom Paarungstyp 2 sind.

13. Zusammensetzung zur herbiziden Kontrolle einer Pflanze der Gattung *Fallopia,* umfassend ein Konsortium von Zellen der Spezies *Mycosphaerella polygoni-cuspidati,* wobei die Zellen im Konsortium alle vom gleichen Paarungstyp sind, wobei die Zusammensetzung weiterhin ein adhäsives Mittel und/oder ein Feuchthaltemittel umfasst.

14. Zusammensetzung nach Anspruch 13, wobei die Zellen Myzelzellen sind.

15. Zusammensetzung nach Anspruch 13 oder 14, wobei die *Mycosphaerellapolygoni-cuspidati*-Zellen im Konsortium alle vom Paarungstyp 1 sind oder alle vom Paarungstyp 2 sind.

## Revendications

1. Utilisation d'un consortium de cellules de l'espèce *Mycosphaerella polygoni-cuspidati* pour la lutte herbicide d'une plante du genre *Fallopia,* dans laquelle les cellules dans le consortium sont toutes du même type de conjugaison.

2. Utilisation selon la revendication 1, dans laquelle la plante est de l'espèce ou hybride: *Fallopia japonica, Fallopia sachalinensis* ou *Fallopia x bohemica,* de préférence *Fallopia japonica* var. *japonica.*

3. Utilisation selon la revendication 1 ou 2, dans laquelle les cellules sont des cellules de mycélium.

4. Utilisation selon l'une quelconque des revendications 1 à 3, dans laquelle les cellules de *Mycosphaerella polygoni-cuspidati* dans le consortium sont toutes du type de conjugaison 1.

5. Utilisation selon l'une quelconque des revendications 1 à 3, dans laquelle les cellules de *Mycosphaerella polygoni-cuspidati* dans le consortium sont toutes du type de conjugaison 2.

6. Procédé pour la lutte herbicide d'une plante du genre *Fallopia,* comprenant la mise en contact de la plante avec une composition comprenant un consortium de cellules de l'espèce de *Mycosphaerella polygoni-cuspidati,* dans lequel les cellules dans le consortium sont toutes du même type de conjugaison.

7. Procédé selon la revendication 6, comprenant en outre l'étape consistant à maintenir les cellules dans un environnement aqueux sur la plante pendant au moins 12 heures, de préférence au moins 24 heures.

8. Procédé selon la revendication 6 ou 7, comprenant la mise en contact des feuilles de la plante avec la composition.

9. Procédé selon l'une quelconque des revendications 6 à 8, dans lequel la plante est de l'espèce ou hybride: *Fallopia japonica, Fallopia sachalinensis* ou *Fallopia x bohemica,* de préférence *Fallopia japonica* var. *japonica.*

10. Procédé selon l'une quelconque des revendications 6 à 9, dans lequel les cellules sont des cellules de mycélium.

11. Procédé selon l'une quelconque des revendications 6 à 10, dans lequel les cellules de *Mycosphaerella polygoni-cuspidati* dans le consortium sont toutes du type de conjugaison 1.

12. Procédé selon l'une quelconque des revendications 6 à 10, dans lequel les cellules de *Mycosphaerella polygoni-cuspidati* dans le consortium sont toutes du type de conjugaison 2.

13. Composition pour la lutte herbicide d'une plante du genre *Fallopia* comprenant un consortium de cellules de l'espèce *Mycosphaerella polygoni-cuspidati,* dans laquelle les cellules dans le consortium sont toutes du même type de conjugaison, la composition comprenant en outre un agent adhésif et/ou un agent mouillant.

14. Composition selon la revendication 13, dans laquelle les cellules sont des cellules de mycélium.

15. Composition selon la revendication 13 ou 14, dans laquelle les cellules de *Mycosphaerella polygoni-cuspidati* dans le consortium sont toutes du type de conjugaison 1 ou sont toutes du type de conjugaison 2.
